# EUROPEAN PATENT SPECIFICATION

(11) **EP 2 658 840 B1**
(45) Date of publication and mention of the grant of the patent: **03.07.2019**
(21) Application number: 10800762.6
(22) Date of filing: 28.12.2010
(51) Int. Cl.: C07C 213/10, C07C 215/28

(54) **PROCESS FOR MAKING FINGOLIMOD HYDROCHLORIDE CRYSTALS**
VERFAHREN ZUR HERSTELLUNG VON FINGOLIMOD-HYDROCHLORIDKRISTALLEN
PROCÉDÉ DE FABRICATION DE CRISTAUX DE CHLORHYDRATE DE FINGOLIMOD

(43) Date of publication of application: 06.11.2013
(73) Proprietor: Synthon BV, 6545 CM Nijmegen (NL)
(72) Inventor: KRAJCOVIC, Jozef, 67817 Blansko (CZ)
(74) Representative: van der Sterren-Mol, Josephine
(86) International application number: PCT/EP2010/070780
(87) International publication number: WO 2012/089238

(56) References cited:
- WO-A1-00/27798
- WO-A1-2012/041707
- WO-A2-2010/055028

## Description

### BACKGROUND OF THE INVENTION

Fingolimod (often coded as FTY 720), chemically 2-amino-2-[2-(4-octylphenyl)ethyl]-propane-1,3-diol of the formula (1) 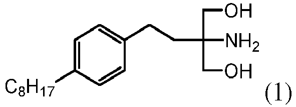 is a pharmaceutically active compound currently tested as an immunosuppressive drug and as an active agent in treatment of multiple sclerosis. It may form stable acid addition salts, of which fingolimod hydrochloride is the most common one.

Fingolimod has been first disclosed in EP 627406 of Yoshitomi, where also two basic routes for making it have been described.

Fingolimod hydrochloride exists in a stable crystalline form. Such crystalline product was first obtained in the above EP 627406 by recrystallization of fingolimod hydrochloride from a solution thereof in ethanol. In Example 5 of WO 2000/027798, crystals of fingolimod hydrochloride were obtained by crystallization from a mixture of ethyl acetate and ethanol. In Example 3 thereof, crystals were obtained by precipitation after concentration of the ethanolic solution. WO2010/055028 discloses the existence of different crystalline forms of fingolimod hydrochloride in particular form I, II and III. It is reported that form I is stable at room temperature and is generally obtained at a temperature below 40°C.

It was found by the present inventor after repeating the crystallization processes of the prior art that fingolimod hydrochloride is obtainable only as a waxy- or a cotton-like solid with poor filterability and flowability. Because of these properties, such crystal habit is inconvenient for large scale processing and for subsequent formulation into pharmaceutical compositions.

Thus, while processes for making crystalline fingolimod hydrochloride are known in the art, an improvement in the matter is still desirable.

### SUMMARY OF THE INVENTION

The present invention is based on a discovery of an improved process for making crystalline fingolimod hydrochloride.

In a first aspect, the present invention provides for a process of making crystalline fingolimod hydrochloride of formula (1a) Form 1, 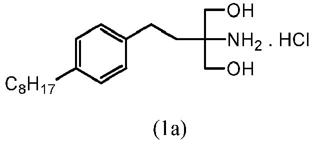 represented by the XRPD pattern 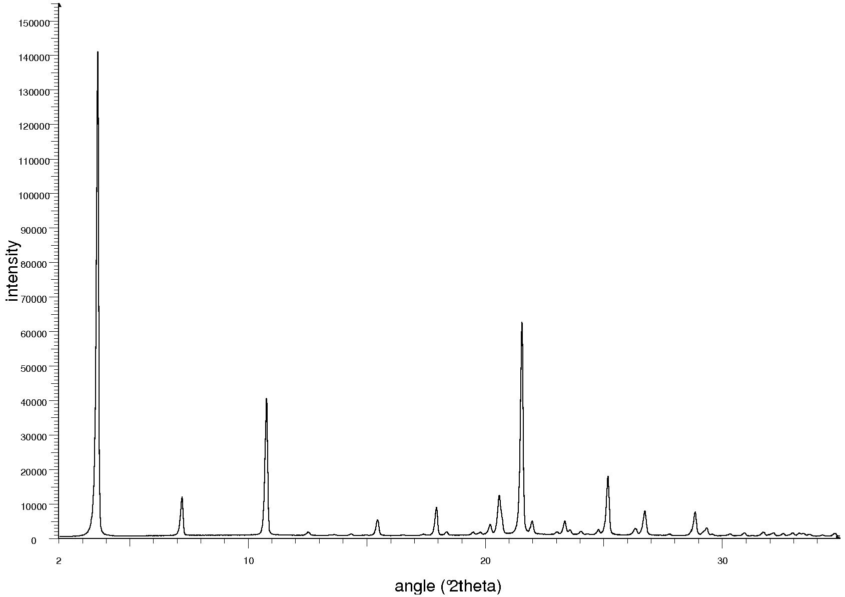 comprising a sequence of steps of
a] contacting a solution of fingolimod hydrochloride in a solvent with an antisolvent at a temperature of at least 50°C wherein the temperature of the antisolvent before and during contacting it with the solvent is at least 40°C,
b] cooling the obtained solution to a temperature of below 40°C, whereby crystalline fingolimod hydrochloride precipitates, and
c] isolating crystalline fingolimod hydrochloride from the mixture,
   wherein
   the solvent is C1-C6 aliphatic alcohol and the antisolvent is n-heptane.

### BRIEF DESCRIPTION OF THE DRAWINGS

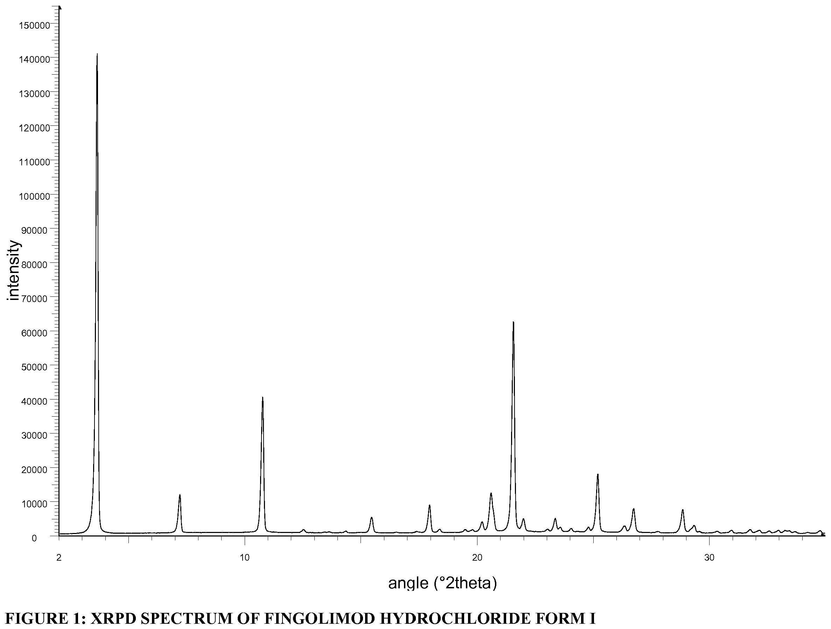

- Fig. 1: XRPD pattern of "Form I" fingolimod hydrochloride

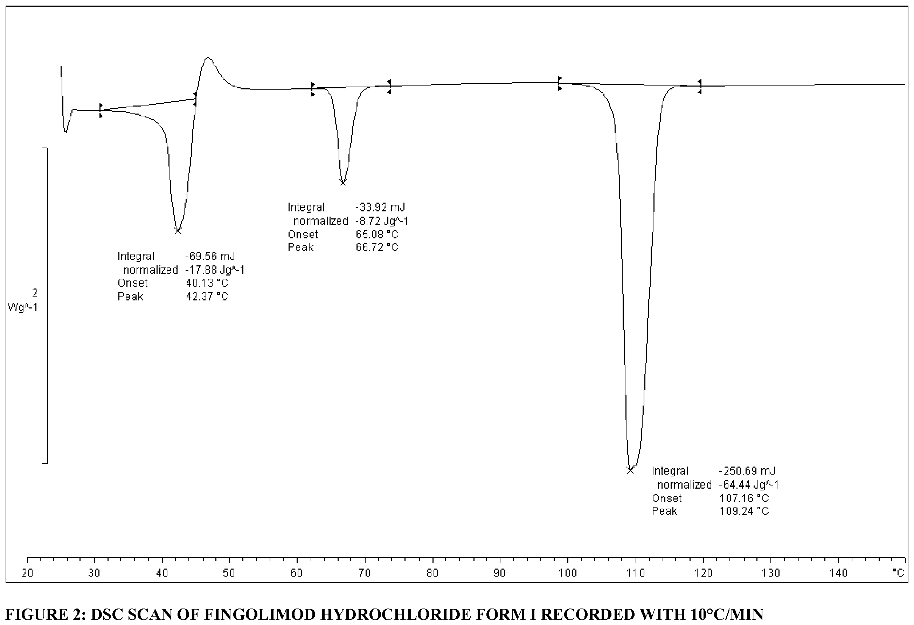

- Fig.2: DSC scan of "Form I" fingolimod hydrochloride

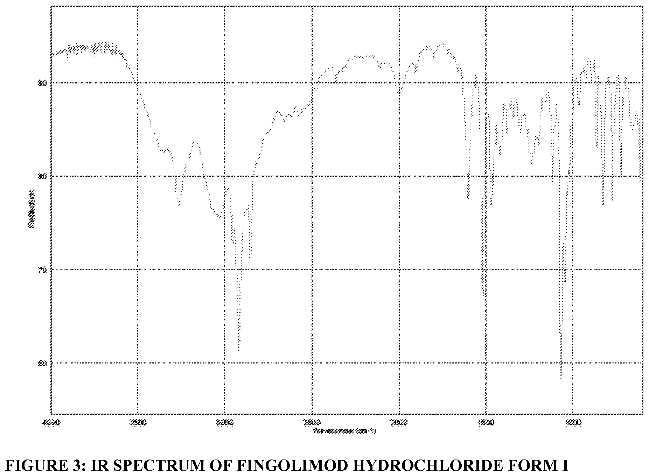

- Fig.3: IR spectrum of Form I" fingolimod hydrochloride

### DETAILED DESCRIPTION OF THE INVENTION

The crystalline fingolimod hydrochloride obtainable by processes of the prior art documents disclosed above is characterized by a distinctive XRPD pattern and IR spectrum, which allow to conclude that each of these processes provides a stable crystalline form, which is denoted for purpose of the present invention as Form I.

The process of our invention provides the same crystalline form, however in a better habit which results in better filterability and flowability. In particular, the population of crystals obtainable by the process of the present invention is characterised by small regular crystals with low tendency to adhere together and to adsorb solvent on the surface of the crystals.

The general feature characterizing the process of the present invention is in that the crystalline fingolimod hydrochloride does not crystallize from an oversaturated solution in a solvent. Instead, crystals of fingolimod hydrochloride are obtained by crystallization from a solution in a mixture of a solvent and an antisolvent (as defined later). Careful selection of the nature of both these liquids as well as of their mutual ratio and of the concentration of fingolimod hydrochloride in the mixture is performed with the goal to obtain a system, which allows to dissolve fingolimod hydrochloride in the mixture at temperatures of at least 50°C, whereby, simultaneously, the same system allows to precipitate fingolimod hydrochloride at temperatures of 40°C and lower. Such a precipitation provides small regular crystals, which exhibit good filterability and flowability after drying. As a result, isolation and elaboration procedures of the solid fingolimod hydrochloride are short, which is advantageous, in particular, at a large scale production process.

The second advantageous feature of the process of the present invention is in that it provides the crystalline Form I defined above with no contamination with other crystalline forms. Careful study of solid state properties of the crystalline fingolimod hydrochloride confirmed the teaching recently disclosed in WO 2010/055028 shown that fingolimod hydrochloride may form metastable crystalline forms upon heating. At least two crystalline forms, hereby denoted as Form II and Form III, respectively, are formed after heating the Form I at 40-60°C or 80°C, resp. Each of the metastable forms converts to the stable Form I after cooling, however, at least in case of the Form II, such conversion is very slow. Thus, precipitating fingolimod hydrochloride at temperatures higher than about 40°C, which is the typical feature of the prior art crystallization procedures, may produce fingolimod hydrochloride as a mixture of crystalline forms, which is undesirable.

The second production possibility known in the art, namely crystallization from a solution that has been concentrated by evaporation, has yet another disadvantage that solvated crystals and/or liquid crystals might be formed in case of too high degree of concentration (such forms have been detected after thorough concentration of solutions of fingolimod hydrochloride in water or in methanol) . Such lyotropic crystal forms are metastable, they however might impurify the desired Form I (and thus alter the physical properties thereof) in case of incomplete removal of the solvent.

In the first technological step of the process of the present invention, a solution of fingolimod hydrochloride in a solvent is provided. The starting fingolimod hydrochloride may preferably be an isolated solid material obtained after separation of the solid from a reaction mixture comprising it. Alternatively, a reaction mixture comprising fingolimod hydrochloride and the solvent (as defined hereinafter) may be used as well. Yet alternatively, fingolimod hydrochloride may be produced directly in the solvent by treatment of fingolimod base with hydrogen chloride.

The "solvent" as used throughout the present invention, is a liquid, in which fingolimod hydrochloride is sufficiently soluble, at least at enhanced temperature. Preferably, the concentration of fingolimod hydrochloride in the solvent at such temperature is higher than 2.5 g/100 ml, advantageously higher than 10g/100 ml.

The solvent of the present invention is an aliphatic alcohol of 1 to 6 carbon atoms, both straight or branched, such as methanol, ethanol, n-propanol, isopropanol, and/or n-butanol.

To the obtained solution, which is kept at the temperature of at least 50°C, the antisolvent is added in such a way that the temperature of the mixture during the mixing step does not drop below 40°C.

The "antisolvent" is an organic liquid, in which fingolimod hydrochloride is essentially insoluble, at least at ambient temperature. Furthermore, the antisolvent is miscible with the solvent. The antisolvent is n-heptane.

Advantageously, the temperature of the antisolvent before and during mixing with the solvent is the same is the solution of fingolimod hydrochloride in the solvent. It is not lower than 40°C. The final amount of the antisolvent is such that the fingolimod hydrochloride is still dissolved in the obtained final mixture. If this is not the case, the temperature of the system must be increased or the mixture must be diluted with the solvent. Useful ratio between the solvent and antisolvent is from 1:2 to 1:20 (v/v), typically about 1:4 to 1:10 (v/v).

After the solution of fingolimod hydrochloride in the solvent/antisolvent mixture is obtained, it is cooled to a temperature of below 40°C and stirred at this temperature, whereby crystals of fingolimod hydrochloride separate from the solution. As shown above, the temperature of 40°C is the critical temperature, above which the crystallization should not occur for to obtain the proper crystalline Form I. The cooled solution may be advantageously seeded with seed crystals of the Form I of fingolimod hydrochloride. In an advantageous mode, the formed suspension of fingolimod hydrochloride in the solvent/antisolvent mixture is stirred for some time, typically from 30 minutes to 2 hours, preferably at ambient or lower than ambient temperature. Further prolongation of stirring has essentially no improving effect (only a decent growth of the originally formed crystals has been sometimes observed).

In the third technological step, the formed crystalline fingolimod hydrochloride is separated from the mother liquor, e.g. by filtration or centrifugation, is washed, advantageously by the antisolvent, and dried until volatile residues, if any, are removed. Further treatment, e.g. milling, is essentially not necessary, but not excluded.

The produced crystalline fingolimod hydrochloride may be used as a pharmaceutically active compound for making pharmaceutical compositions for treatment various diseases, as known in the art.

The present invention is illustrated by following examples.

### EXAMPLES

### Comparative example 1 (example 29 of EP 0627406 B1)

1.0 g of fingolimod hydrochloride was dissolved in 3 ml of ethanol at reflux. The solution was allowed to cool to room temperature and stirred at room temperature for about 1 hour. As a result, a solid was formed (thick cake). The solid was isolated by filtration over a P3-glass filter (reduced pressure) and air-dried overnight at R.T. An off-white, greasy to sticky solid with lumps was obtained. The yield was 0.70 g (70%).
DSC: solid-solid transitions below 75°C and melting around 107-110°C.

### Comparative example 2 (example 5 of WO 2000/27798)

1.0 g of fingolimod hydrochloride was dissolved in 4 ml of ethanol/ethyl acetate (1:1 V/V) at reflux. The solution was stirred and allowed to cool to room temperature. As a result, a solid was formed (thick cake, sudden crystallization). The solid was isolated by filtration over a P3-glass filter (reduced pressure) and air-dried overnight at R.T. An off-white, greasy to sticky solid with lumps was obtained. The yield was 0.86 g (86%).
DSC: Similar to the DSC scan of Comparative example 1.

### Example 1: Precipitation of fingolimod hydrochloride Form I crystals

Fingolimod (5 g, 16.1 mmol) was stirred with 2-propanol (30 ml). Into the heterogenic mixture, hydrogen chloride solution in 2-propanol (21%) (3.7 ml) was dropwise added. The heterogeneous mixture was warmed to 50°C. Into resulting clear solution, pre-heated n-heptane (120 ml) was added slowly, while the homogeneity of the reaction mixture was maintained. Clear reaction mixture was stirred at a temperature of 50°C for 10 min. The solution was cooled to 0°C during 15 min. The resulted suspension was stirred at 0-3°C for 40 min, filtered off, washed with 2 x 15 ml n-heptane. The yield of fingolimod hydrochloride was 5.1 g (91%) of off-white crystals.

## Claims

1. A process of making crystalline fingolimod hydrochloride Form 1 of formula (1a), 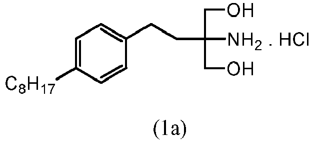 represented by the XRPD pattern 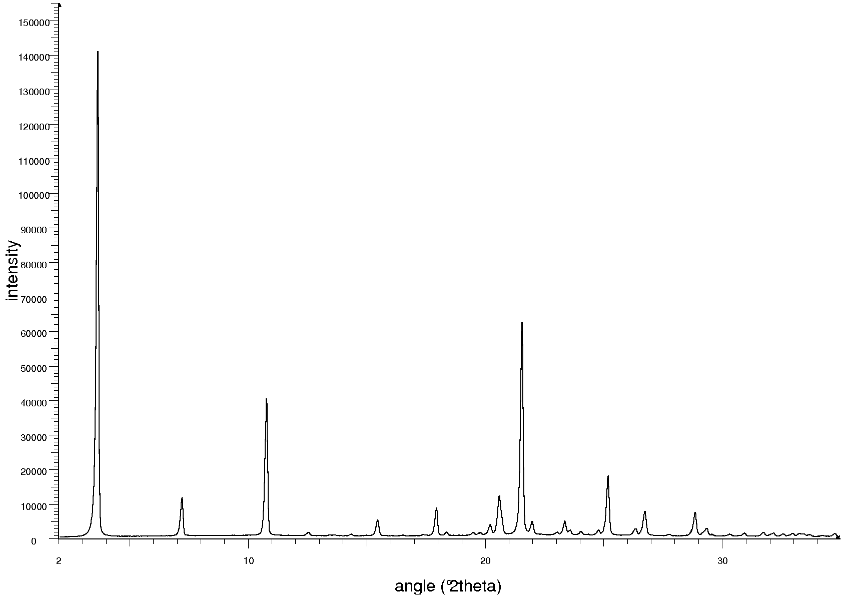 comprising a sequence of steps of
a] contacting a solution of fingolimod hydrochloride in a solvent with an antisolvent at a temperature of at least 50°C wherein the temperature of the antisolvent before and during contacting it with the solvent is at least 40°C,
b] cooling the obtained solution to a temperature of below 40°C, whereby crystalline fingolimod hydrochloride precipitates, and
c] isolating crystalline fingolimod hydrochloride from the mixture,
wherein
the solvent is a C₁-C₆ aliphatic alcohol and the antisolvent is n-heptane.

2. The process according to claim 1, wherein the solvent is selected from the group comprising methanol, ethanol, n-propanol, isopropanol, and/or n-butanol, and mixtures thereof with water.

3. The process according to claim 1 or 2, wherein the concentration of fingolimod hydrochloride in the solvent is higher than 2.5 g/100 ml.

4. The process according to claim 1 or 2, wherein the concentration of fingolimod hydrochloride in the solvent is higher than 10 g/100 ml.

5. The process according to any one of claims 1 to 4, wherein the ratio between the solvent and antisolvent is from 1:2 to 1:20 (v/v).

6. The process according to any one of claims 1 to 4, wherein the ratio between the solvent and antisolvent is from 1:4 to 1:10 (v/v)

7. The process according to any one of claims 1 to 6, wherein the formed suspension in the solvent/antisolvent mixture is stirred after completing the precipitation process for 30 minutes to 2 hours.

## Patentansprüche

1. Prozess zur Herstellung von kristallinem Fingolimod-Hydrochlorid Form 1 nach der Formel (1a), 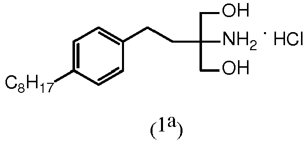 dargestellt durch das Röntgenbeugungsmuster im Pulver 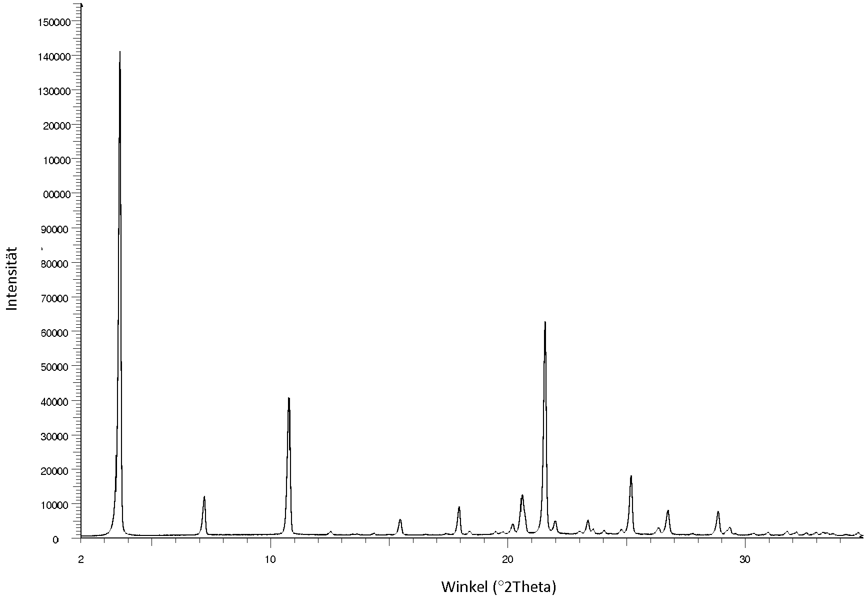 eine Folge von Schritten umfassend:
a] Inkontaktbringen einer Lösung von Fingolimod-Hydrochlorid in einem Lösungsmittel mit einem Antilösungsmittel bei einer Temperatur von mindestens 50 °C, wobei die Temperatur des Antilösungsmittels vor und nach dem Inkontaktbringen mit dem Lösungsmittel zumindest 40 °C beträgt,
b] Kühlen der erhaltenen Lösung auf eine Temperatur von unter 40 °C, wobei kristallines Fingolimod-Hydrochlorid ausfällt, und
c] Isolieren des kristallinen Fingolimod-Hydrochlorids aus der Mischung,
wobei
das Lösungsmittel ein C₁-C₆-aliphatischer Alkohol und das Antilösungsmittel n-Heptan ist.

2. Prozess nach Anspruch 1, wobei das Lösungsmittel aus der Gruppe ausgewählt wird, die Methanol, Ethanol, n-Propanol, Isopropanol und/oder n-Butanol und Mischungen davon mit Wasser umfasst.

3. Prozess nach Anspruch 1 oder 2, wobei die Konzentration von Fingolimod-Hydrochlorid im Lösungsmittel mehr als 2.5 g/100 ml beträgt.

4. Prozess nach Anspruch 1 oder 2, wobei die Konzentration von Fingolimod-Hydrochlorid im Lösungsmittel mehr als 10 g/100 ml beträgt.

5. Prozess nach einem der Ansprüche 1 bis 4, wobei das Verhältnis von Lösungsmittel und Antilösungsmittel zwischen 1:2 und 1:20 (v/v) beträgt.

6. Prozess nach einem der Ansprüche 1 bis 4, wobei das Verhältnis von Lösungsmittel und Antilösungsmittel zwischen 1:4 und 1:10 (v/v) beträgt.

7. Prozess nach einem der Ansprüche 1 bis 6, wobei die in der Lösungsmittel/Antilösungsmittel-Mischung gebildete Suspension nach Abschluss des Fällungsprozesses für zwischen 30 Minuten und 2 Stunden gerührt wird.

## Revendications

1. Processus de fabrication du chlorhydrate de fingolimod cristallin au Formulaire 1 avec la formule (1a), 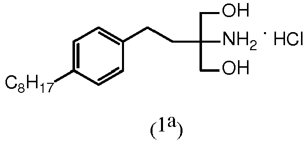 représentée par le schéma XRPD 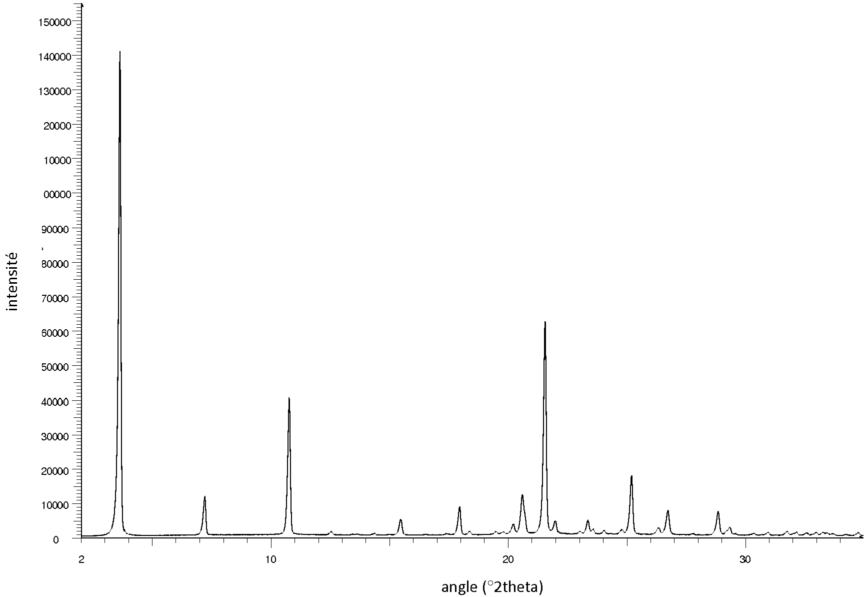 comprenant une séquence d'étapes consistant à
a] mettre en contact une solution de chlorhydrate de fingolimod dans un solvant avec un antisolvant à une température d'au moins 50 °C dans laquelle la température de l'antisolvant avant et pendant le contact avec le solvant est d'au moins 40 °C,
b] refroidir la solution obtenue à une température inférieure à 40 °C, dans laquelle le chlorhydrate de fingolimod cristallin forme un précipité, et
c] isoler le chlorhydrate de fingolimod cristallin du mélange,
dans lequel
le solvant est un alcool aliphatique C₁- C₆ et l'antisolvant est le n-heptane.

2. Procédé selon la revendication 1, dans lequel le solvant est choisi dans le groupe comprenant du méthanol, de l'éthanol, du n-propanol, de l'isopropanol et/ou du n-butanol, et des mélanges de ceux-ci avec de l'eau.

3. Procédé selon la revendication 1 ou 2, dans lequel la concentration du chlorhydrate de fingolimod dans le solvant est supérieure à 2.5 g/100 ml.

4. Procédé selon la revendication 1 ou 2, dans lequel la concentration du chlorhydrate de fingolimod dans le solvant est supérieure à 10 g/100 ml.

5. Procédé selon l'une quelconque des revendications 1 à 4, dans lequel le rapport entre le solvant et l'antisolvant est de 1:2 à 1:20 (v/v).

6. Procédé selon l'une quelconque des revendications 1 à 4, dans lequel le rapport entre le solvant et l'antisolvant est de 1:4 à 1:10 (v/v)

7. Procédé selon l'une quelconque des revendications 1 à 6, dans lequel la suspension formée dans le mélange solvant/antisolvant est remuée après avoir terminé le processus de précipitation pendant 30 minutes à 2 heures.
